(19) Europäisches Patentamt
European Patent Office
Office européen des brevets

(11) Publication number: 0 022 164
B1

(12) **EUROPEAN PATENT SPECIFICATION**

(45) Date of publication of patent specification: 12.01.83

(51) Int. Cl.³: C 07 C 33/046, C 07 C 29/00, B 01 J 31/06

(21) Application number: 80102863.0

(22) Date of filing: 22.05.80

(54) **A process for the preparation of 1,4-butynediol and related catalyst.**

(30) Priority: 15.06.79 IT 2365679

(43) Date of publication of application:
14.01.81 Bulletin 81/2

(45) Publication of the grant of the patent:
12.01.83 Bulletin 83/2

(84) Designated Contracting States:
DE FR GB SE

(56) References cited:
DE - B - 2 429 269

Chemical Abstracts vol. 84, no. 26, 1976 Columbus, Ohio, USA E. TSUCHIDA et al. "Effect of nonpolar field formed by polymer ligand on the oxidation of 2.6-xylenol catalyzed by copper complexes" page 6, column 1, abstract no. 180726g

INDUSTRIAL AND ENGINEERING CHEMISTRY. PRODUCT RESEARCH AND DEVELOPMENT, Vol. 17, No. 2, 1978 Columbus, Ohio, USA H. HAYASHI "Oxidative Coupling of Diphenylmethanimine Catalyzed by Copper-Loaded Copolymers of 4-Vinylpyridine and Divinylbenzene" pages 128 to 133

(73) Proprietor: SISAS Società Italiana Serie Acetica Sintetica S.p.A.
Largo Corsia dei Servi 3
I-20122 Milano (IT)

(72) Inventor: Codignola, Franco
Via Zanardelli 31
I-25014 Castenedolo Brescia (IT)
Inventor: Gronchi, Paolo
Via Servio Tulio 4
I-20100 Milano (IT)
Inventor: Vergini, Giorgio
Via Venini 49
I-20100 Milano (IT)
Inventor: Dell Rosso, Renato
Via Palmanova 67
I-20132 Milano (IT)

(74) Representative: Bühling, Gerhard, Dipl.-Chem. et al,
Patentanwaltsbüro Tiedtke-Bühling-Kinne-Grupe-Pellmann-Dragotti Bavariaring 4
D-8000 München 2 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 022 164 B1

# A process for the preparation of 1,4-butynediol and related catalyst

The present invention relates to an improved process for the manufacturing of 1,4-butynediol, and to the related catalyst. 1,4-Butynediol has been and is today used as an intermediate for the production of butadiene: 1,4-butynediol is hydrogenated to 1,4-butanediol, from which butadiene is obtained through bilateral dehydration.

Furthermore, 1,4-butanediol too has recently found an increasing use for the production of saturated polyester resins (especially PBT), of expanded and polymeric polyurethane resins and in the production of tetrahydrofuran by monodehydration.

The typical reaction scheme for the production of 1,4-butynediol is illustrated by the following reactions:

$$1)\ HC\equiv CH + HCHO \xrightarrow{CuC_2} HC\equiv C—CHOH$$

$$2)\ HC\equiv C—CH_2OH + HCHO \xrightarrow{CuC_2} HOCH_2—C\equiv C—CH_2OH$$

Such a synthesis was disclosed for the first time, from the industrial point of view, in the US—A 2,232,867 to Reppe et al; more particularly, as the catalyst, monovalent copper supported onto an inorganic polymer $(SiO_2)_n$ was indicated, the catalyst being prepared by impregnation and subsequent calcination. According to the preferred modification of this process, besides the monovalent copper, the catalyst was also impregnated with a small percentage of bismuth, having the purpose of enhancing the selectivity of the catalyst and inhibiting the polymerization of acetylene to cuprene. This is a very important feature, since the catalyst becomes coated with cuprene, thus inhibiting the catalytic function and clogging the catalyst bed, thus causing self-evident problems.

Thereafter several other supports for the active metal have been proposed, such as carbon, pumice, magnesium silicates, alumina, or inorganic mixtures of magnesium and aluminium salts and/or oxides.

It is to be noted that according to the process disclosed in the above numbered U.S. patent (Reppe et al) and the industrial processes as carried out to date, the reaction was effected under pressure of about 4.9 to 6.9 bar and at a temperature of between 90 and 95°C. The fact should not be neglected that, under the above pressures, particular cautions must be adopted, in order to prevent acetylene from undergoing explosive decomposition.

Moreover, according to the industrial process as used to date, by operating under the above indicated conditions and with a catalyst containing at least 12% copper and at least 6% bismuth, production rates were obtained (as reported in the literature) of about 1 kg 1,4 butynediol/1kg catalyst/24 hours operation.

The main purpose of the present invention is that of providing a process for the production of 1,4-butynediol according to which:

1) the activity and the selectivity of the catalyst are improved, thus increasing the production rate, and enabling the reaction to be carried out under pressure and temperature conditions definitely lower than the dangerous limits as regards the explosive decomposition of acetylene;

(2) the formation of cuprene is essentially eliminated, whereby the catalytic bed and the catalyst activity are essentially safeguarded.

It has been now found, which is the subject of the present invention, that these purposes are essentially achieved by means of a process for the preparation of 1,4-butynediol, wherein acetylene is reacted with formaldehyde in the presence of a metal catalyst, particularly and preferably copper supported onto an inert support, characterized in that a vinylpyridine polymer matrix is used as the catalyst support, said polymer being complexed with the catalytic metal. According to the preferred embodiment, said vinylpyridine polymer is a copolymer of the vinylpyridine with a comonomer selected from vinylaromatic and vinylaliphatic monomers. According to an alternative embodiment of the present invention said support of the metal comprises a vinylpyridine homopolymer, said support being complexed with the said catalytic metal and being conveniently swelled with a suitable solvent before and/or during the reaction. In both the embodiments of the invention the above reaction is effected at a temperature of between 70 and 150°C (preferably between 78 and 100°C) and at a pressure not higher than 1.47 bar, higher pressures being however possible if due precautions are taken. Considering now more specifically the several parameters of the process of the present invention, it is clear that the relevant and highly surprising advantages of the invention are a consequence of the catalyst used for the reaction and particularly of the support complexed with the catalytic metal. It is necessary to point out that polymers of 4-vinylpyridine were already proposed in the past as catalyst supports (E. M. Cermia, M. Graziani, J. Applied Polymer Science, Vol. 13, page 2742 (1974), "Alcohol Carbonylation", and Ind. Eng. Chem. Prod. Res. Dev. Vol. 17, No. 2 (1978) page 128, "Oxidative

Coupling of Diphenylmethanimine Catalyzed by Copper Loaded Copolymers of 4-Vinylpyridine and DVB)".

However, in all these cases the possibility of employing these catalysts supports in the reaction of the Reppe process has never been suggested.

Furthermore both, the high increase of production rate, as noted in the process of the present invention (which shall be confirmed by the following examples), and the essential elimination of cuprene formation were completely unpredictable.

By the polymers (namely homopolymers and copolymers) of 4-vinylpyridine to be used in the present invention, there are meant those having a molecular weight of at least 5,000 and preferably of between 30,000 and 50,000.

A possible explanation, although without limiting meaning, of the operating mechanism of the catalyst used in the process of the present invention is that the catalytic metal is complexed by the electron donor group containing polymeric molecules, these groups in the case of the copolymers being separated by non-donor groups.

These electron donor groups form the active sites which are responsible for the complexation of the catalytic metal. Thus, by varying the swelling (in the case of homopolymer) or the number of comonomer molecules separating electron donor groups, it is possible to space the atoms of the catalytic metal from each other so that the catalytic action promoting the cuprene formation is made statistically unlikely. At the same time, due to the macroporous or swelled nature of the support polymer, the surface area of the support available for the catalytic metal is increased. This greatly improves the activity and selectivity of the catalyst due to the easier and/or more probable access of reactants to atoms of the catalytic metal which otherwise would remain unused.

In the case of the homopolymers of vinylpyridine, the solvent used for the swelling of the polymer support is, as a rule, inert with respect to the components of the reaction mixture, particularly a polar solvent. Preferably this solvent is selected from aliphatic, alicyclic and cyclic alcohols.

Turning lastly to the pressure conditions under which the reaction of the present invention is effected, it is to be pointed out that, thanks to the use of the afore said catalyst, these pressures, with particular reference to the acetylene, are definitely lower than those of the processes used to date and always lower than those of the explosive dangerous limit, of pure acetylene.

Whilst, according to the prior art processes, acetylene pressures were used of 4.9 to 6.9 bar, namely higher than the explosive limit, in the process of the invention the maximum pressure of acetylene, fed at a relative pressure of 1.47 bar, within the reaction vessel is of 693 mbar gauge, which means less than the explosive limit.

If account is taken of the fact that the higher is the reaction temperature, the higher is the absolute pressure at which the reaction takes place, the fact that in the present invention the partial pressure of acetylene in the reaction environnment is at maximum 693 mbar is even more surprising, since the reaction temperature in the present invention is equal or even little higher than that of the prior art processes.

For a comparison with the dangerous levels indicated in the literature, see S. A. Miller, "Acetylene", E. Bems Edit. Ltd. London 1945, chap. 6, page 476.

A further advantage connected with the use of relatively low acetylene pressures is that it tends to reduce the formation of acetylene polymers and cuprenes, the importance of which as regards the process and particularly as regards the useful life of the catalyst having been discussed above.

Of course, the use of higher pressures of acetylene still comes within the scope of the present invention.

The following examples are given only to illustrate the invention, and should not be construed as limiting the scope of the invention.

The homopolymer and the copolymer, cross-linked with divinylbenzene, wer supplied by CRODA Synthetic Chemicals Ltd. The gas-chromatographic analyses were carried out by means of a Perkin-Elmer Sigma 3 chromatographic apparatus, having a Sigma 10 computer, by using a 1m long glass column, packed with Carbowax® 3% on Cromosorb, of the diameter of 3 mm. The analyses of the copper present on the catalyst were carried out according to the iodometric method (ASTM 34—68), and those relating to the formaldehyde according to the $Na_2SO_3$ method.

The preparation of the catalytic complexes was effected by using the normal techniques, well known in the art.

Example 1

In an oscillating steel autoclave equipped with an acetylene entrance cock and a thermometric well for the temperature control, the autoclave having a capacity of 320 ml and being heated by means of an electrical resistance, there are charged 4.8 g of the complex containing 20% Cu coordinated with the ligands contained in the polymer matrix of the 4-vinylpyridine homopolymer, and 180 ml of a 30% HCHO solution (486 g of HCHO) in isobutanol. Oxygen is eliminated from the reaction environment according to the normal technique of several purging passes of pure nitrogen.

Before starting the heating and the stirring, a vacuum of about 0.1 bar (abs.) is established in the autoclave. When the temperature has attained 120°C, $C_2H_2$ is fed up to 2.45 bar (abs.). The absolute

pressure of $C_2H_2$ in this case is 1.317 bar and thus lower than the dangerous limit. After the 4 hours needed for catalyst formation, the monitoring of the production rate is commenced by gas-chromatographic analysis of small samples taken each hour for the subsequent six hours.

An average production rate of propargyl alcohol of 0.015 g/ 1g of catalyst × 1 h and of 1,4-butynediol of 0.1 g/1g of catalyst ×1 h is obtained.

The following examples were carried out in glass autoclaves having a capacity of 500 ml, an acetylene entrance cock and a further tap connected to an equipment for establishing a vacuum without the autoclave.

The latter was heated by a thermostatic oil bath at the temperature of 95°C, and the internal temperature was determined by means of a thermocouple placed in a thermowell. The reaction mixture was stirred by means of a magnetic stirrer.

Example 2

The autoclave is charged with 200 ml of a 36% solution of HCHO (79g) in water and with 10 g of the Cu complex (Cu 4.1%), the ligands being part of a polymeric matrix comprising 4-vinylpyridine (60% by weight) and divinylbenzene (40%). Acetylene is continuously fed through a pipe dipped into the solution.

The partial pressure of acetylene is 686 mbar and thus definitely less than the dangerous limit.

After 4 hours time necessary for catalyst formation, the monitoring of the production rate is commenced, using gas-chromatographic examinations of small samples taken every hour. The results indicated a production rate of propargyl alcohol of 0.024 g/1g catalyst × 1H and of 1,4-butynediol of 0.06 g/1g catalyst × 1h.

Example 3

Under the same temperature and pressure conditions [90°C internal temperature and 2.45 bar (abs.) of $C_2H_2$ feed pressure], the reaction is carried out with 10 g of a complex containing 3% Cu coordinated with the ligand groups of a polymeric matrix comprising 4-vinylpyridine (94% by weight) and divinylbenzene (4%), and 200 ml of a 36% HCHO solution.

Under conditions of steady operation there are obtained 0.017 g/1g catalyst × 1 h of propargyl alcohol and 0.23 g/1g catalyst × 1 h of 1,4-butynediol.

In the preceding examples reference has been made to the catalyst of metal copper supported on a polymeric matrix, but the same polymeric support is suitable as well for any other catalytic metal adapted to form with acetylene, under the specific reaction conditions, the corresponding acetylides, such as for example Ni, Hg, Au or Pd.

**Claims**

1. A process for the preparation of 1,4-butynediol, wherein acetylene and formaldehyde are reacted in the presence of a catalytic metal selected from Cu, Ni, Hg, Au and Pd which is capable of forming, under the reaction conditions, acetylides with the acetylene, characterized in that the catalytic metal is complexed with a polymeric matrix formed by a polymer of 4-vinylpyridine.

2. A process for the preparation of 1,4-butynediol according to claim 1, characterized in that said polymer of the 4-vinylpyridine has a molecular weight of at least 5,000, preferably of between 30,000 and 50,000.

3. A process for the preparation of 1,4-butynediol according to claim 1, characterized in that said polymer of the 4-vinylpyridine is a copolymer comprising comonomers, grafted and/or cross-linked to the vinylpyridine chain, said comonomers being selected from vinylaliphatic and vinylaromatic comonomers.

4. A process for the preparation of 1,4-butynediol according to claim 1, characterized in that the catalytic metal is copper.

5. A process for the preparation of 1,4-butynediol according to claim 1, characterized in that said reaction is effected at a temperature of between 70°C and 150°C.

6. A process for the preparation of 1,4-butynediol according to claim 5, characterized in that said reaction temperature is between 80°C and 100°C.

7. A process for the preparation of 1,4-butynediol according to claim 1, characterized in that said reaction is carried out at a pressure lower than the limit of explosive decompositon of acetylene under the thermal conditions of the reaction.

8. A process for the preparation of 1,4-butynediol according to the preceding claims, characterized in that the partial pressure of the acetylene fed to the reactor is not higher than 1.47 bar.

9. Catalyst for the preparation of 1,4-butynediol by reacting acetylene and formaldehyde according to the process of the preceding claims, characterized in that it is a polymer of 4-vinylpyridine complexed with copper.

10. Catalyst according to claim 9, characterized in that said polymer of vinylpyridine is a homopolymer having a molecular weight of at least 5,000 and swelled with a solvent which, under the reaction conditions, in inert with respect to the components of the reaction mixture.

11. Catalyst according to claim 9, characterized in that said polymer is a copolymer of 4-vinylpyridine with a comonomer selected among vinylaliphatic and vinylaromatic monomers.

**Revendications**

1. Un procédé pour la préparation de 1,4-butynediol, dans lequel acétyléne et formaldéhyde sont faites réagir en présence d'un métal catalytique choisi parmi Cu, Ni, Hg, Au et Pd qui est à même de former dans des conditions de réaction acétylures avec l'acetyléne, caractérisé par le fait que le métal catalytique est complexé avec une matrice polymérique formée par un polymére de 4-vinylpyridine.

2. Un procédé pour la préparation de 1,4-butynediol selon la révendication 1, caractérisé par le fait que le dit polymére de 4-vynil pyridine a un poids moléculaire d'au moins 5.000, préferablement entre 30.000 et 50.000.

3. Un procédé pour la préparation de 1,4-butynediol selon la révendication 1, caractérisé par le fait que le dit polymére de 4-vinyl pyridine est un polymére comprenant des comonoméres greffés et/ou "cross-linked" à la chaine de polyvinyl pyridine: les dits comonoméres sont selectionés parmi des comonoméres vinylaliphatiques vinylaromatiques.

4. Un procédé pour la préparation de 1,4-butynediol selon la révendication 1, caractérisé par le fait que le métal catalytique est le cuivre.

5. Un procédé pour la préparation de 1,4-butynediol selon la révendication 1, caractérisé par le fait que la dite réaction a lieu à une température entre 70 et 150°C.

6. Un procédé pour la préparation de 1,4-butynediol selon la révendication 5, caractérisé par le fait que dans cette réaction la température est entre 80 et 100°C.

7. Un procédé pour la préparation de 1,4-butynediol selon la révendication 1, caractérisé par le fait que cette réaction a lieu à une pression inférieure à la limite de décomposition explosive de l'acétyléne aux conditions thermiques de réaction.

8. Un procédé pour la préparation de 1,4-butynediol selon les révendication précédentes, caractérisé par le fait que la pression partielle de l'acétyléne n'est pas plus haute de 1,47 bar.

9. Catalyseur pour la préparation de 1.4-butynediol en faisant réagir acétylene et formaldéhyde selon le procédé mentionné dans les révendications précédente, caractérisé par le fait qu'il est un polymére de 4-vinyl pyridine complexée avec du cuivre.

10. Catalysueur selon la révendication 9, caractérisé par le fait que le dit polymére de vinyl pyridine est un homopolymére ayant un poids moléculaire d'au moins 5.000 et gonflè avec un solvent qui, aux conditions de réaction, est inerte vis à vis des composants du mélange de réaction.

11. Catalysueur selonla révendication 9, caractérisé par le fait que le dit polymére est un copolymére de la 4-vinylpyridine avec un comonomére selectionné parmi les monoméres vinyl aliphatiques et vinyl aromatiques.

**Patentansprüche**

1. Verfahren zur Herstellung von 1,4-Butindiol, bei dem Acetylen und Formaldehyd in Gegenwart eines aus Cu, Ni, Hg, Au und Pd ausgewählten, katalytischen Metalls, das dazu befähigt ist, unter den Reaktionsbedingungen mit dem Acetylen Acetylide zu bilden, zur Reaktion gebracht werden, dadurch gekennzeichnet, daß das katalytische Metall in einen Komplex mit einer polymeren Matrix, die durch eine Polymeres von 4-Vinylpyridin gebildet wird, übergeführt wird.

2. Verfahren zur Herstellung von 1,4-Butindiol nach Anspruch 1, dadurch gekennzeichnet, daß das Polymere von 4-Vinylpyridin ein Molekulargewicht hat, das mindestens 5000 beträgt und vorzugsweise zwischen 30.000 und 50.000 liegt.

3. Verfahren zur Herstellung von 1,4-Butindiol nach Anspruch 1, dadurch gekennzeichnet, daß das Polymere von 4-Vinylpyridin ein Copolymeres ist, das auf die Vinylpyridinkette aufgeproftе und/oder mit der Vinylpyridinkette vernetzte, aus vinylaliphatischen und vinylaromatischen Comonomeren ausgewählt Comonomere aufweist.

4. Verfahren zur Herstellung von 1,4-Butindiol nach Anspruch 1, dadurch gekennzeichnet, daß das katalytische Metall Kupfer ist.

5. Verfahren zur Herstellung von 1,4-Butindiol nach Anspruch 1, dadurch gekennzeichnet, daß die Reaktion bei einer Temperatur zwischen 70°C und 150°C durchgeführt wird.

6. Verfahren zur Herstellung von 1,4-Butindiol nach Anspruch 5, dadurch gekennzeichnet, daß die Reaktionstemperatur zwischen 80°C und 100°C liegt.

7. Verfahren zur Herstellung von 1,4-Butindiol nach Anspruch 1, dadurch gekennzeichnet, daß die Reaktion bei einem Druck durchgeführt wird, der niedriger ist als der Grenzwert des explosiven Zerfalls von Acetylen unter den thermischen Bedingungen der Reaktion.

8. Verfahren zur Herstellung von 1,4-Butindiol nach den vorhergehenden Ansprüchen, dadurch gekennzeichnet, daß der Partialdruck des dem Reaktionsbehälter zugeführten Acetylens nicht höher als 1,47 bar ist.

9. Katalysator für die Herstellung von 1,4-Butindiol durch Umsetzung von Acetylen und Formaldehyd nach dem Verfahren gemäß den vorhergehenden Ansprüchen, dadurch gekennzeichnet, daß der

Katalysator ein in einen Komplex mit Kupfer übergeführtes Polymeres von 4-Vinylpyridin ist.

10. Katalysator nach Anspruch 9, dadurch gekennzeichnet, daß das Polymere von Vinylpyridin ein Homopolymeres mit einem Molekulargewicht von mindestens 5.000 ist und mit einem Lösungsmittel, das unter den Reaktionsbedingungen gegenüber den Bestandteilen der Reaktionsmischung inert ist, aufgequollen ist.

11. Katalysator nach Anspruch 9, dadurch gekennzeichnet, daß das Polymere ein Copolymeres von 4-Vinylpyridin mit einem aus vinylaliphatischen und vinylaromatischen Monomeren ausgewählten Comonomeren ist.